# EUROPEAN PATENT SPECIFICATION

(11) **EP 2 616 433 B1**
(45) Date of publication and mention of the grant of the patent: **09.12.2015**
(21) Application number: 11758183.5
(22) Date of filing: 13.09.2011
(51) Int. Cl.: C07C 253/30, C07C 51/06, C07C 51/08, C07C 51/60, C07C 61/08, C07C 319/06, C07C 319/22, C07C 327/06, C07C 255/46, C07C 321/28

(54) **PROCESS FOR PREPARING A CYCLOHEXANECARBONITRILE DERIVATIVE**
VERFAHREN ZUR HERSTELLUNG VON CYCLOHEXANCARBONITRILDERIVATEN
PROCEDE DE DERIVES DU CYCLOHEXANECARBONITRILE

(30) Priority: 16.09.2010 EP 10177187
(43) Date of publication of application: 24.07.2013
(73) Proprietor: F.Hoffmann-La Roche AG, 4070 Basel (CH)
(72) Inventor: HARNETT, Gerard, John, Ennis County Clare (IE); HAYES, John, Ennis County Clare (IE); REENTS, Reinhard, CH-4142 Muenchenstein (CH); SMITH, Dennis, A., Ennis County Clare (IE); WALSH, Andrew, Lissycasey County Clare (IE)
(74) Representative: Schirlin, Julien
(86) International application number: PCT/EP2011/065860
(87) International publication number: WO 2012/035017

(56) References cited:
- WO-A1-2009/121788
- WO-A1-2009/137294
- WO-A2-2006/118955
- FLEMING FRASER F ET AL: "Metalated nitriles: Organolithium, -magnesium, and -copper exchange of .alpha.-halo nitriles", JOURNAL OF ORGANIC CHEMISTRY, AMERICAN CHEMICAL SOCIETY, EASTON.; US, vol. 70, no. 6, 1 January 2005 (2005-01-01), pages 2200-2205, XP002494612, ISSN: 0022-3263, DOI: DOI:10.1021/JO047877R [retrieved on 2005-02-22]

## Description

The present invention relates to a process for the preparation of a cyclohexanecarboxylic acid derivative which is useful as an intermediate in the preparation of pharmaceutically active compounds.

In a first embodiment, the present invention provides a process for the preparation of the compound of a cyclohexanecarbonitrile derivative of formula (I): 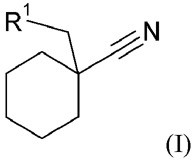 wherein R¹ is (C₁-C₈)alkyl, preferably pent-3-yl, comprising adding a Grignard reagent, such as (C₁-C₆)alkyl-magnesium-halide, phenyl-magnesium-halide, heteroaryl-magnesium-halide or (C₃-C₆)cycloakyl-magnesium-halide to cyclohexanecarbonitrile of formula (II) 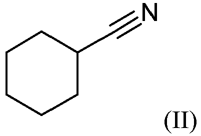 in the presence of an alkylating agent, wherein the alkylating agent is 1-halo-CH₂R¹, preferably 1-halo-2-ethylbutane, or a sulfonate ester of R¹CH₂-OH, preferably of 2-ethyl-1-butanol, wherein R¹is as defined above. The above mentioned coupling reaction is carried out in the presence of a secondary amine. 0.01 to 0.5 equivalents of the secondary amine with respect to cyclohexanecarbonitrile is used.

In particular, the above mentioned coupling reaction is followed by a mineral acid quenching, such as hydrofluoric acid, hydrochloric acid, boric acid, acetic acid, formic acid, nitric acid, phosphoric acid or sulfuric acid, most preferably by hydrochloric acid.

Contrary to expectation it was surprisingly found that adding the Grignard reagent to a mixture of the cyclohexanecarbonitrile and the alkylating agent, instead of first combining the Grignard reagent and the cyclohexanecarbonitrile before coupling with the alkylating agent, led to improved yields and a reduction in the formation of by-products. It is most surprising that the reaction is not complicated by reaction between the Grignard reagent and the alkylating agent.

The compound of formula (I) may be used as intermediate in the synthesis of valuable pharmaceutical compounds. For example 1-(2-ethylbutyl)cyclohexanecarbonitrile may be used in the synthesis of the ones as described in EP 1,020, 439 based on the intermediate process disclosed in WO 2009121788.

Unless otherwise stated, the following terms used in the specification and claims have the meanings given below:
The term "halo" means fluoro, chloro, bromo or iodo, preferably chloro or bromo.
"alkali metal" or "alkali" refers to lithium, sodium, potassium, rubidium and caesium. Preferable alkali metal is lithium or sodium. Of these, sodium is most preferred.
"(C₁-C₈)alkyl" refers to a branched or straight hydrocarbon chain of one to eight carbon atoms, such as methyl, ethyl, n-propyl, isopropyl, n-butyl, isobutyl, sec-butyl, t-butyl, pentyl, hexyl and heptyl. (C₁-C₆)alkyl is preferred.
"(C₁-C₆)alkoxy" means a moiety of the formula -OR^{ab}, wherein R^{ab} is an (C₁-C₆)alkyl moiety as defined herein. Examples of alkoxy moieties include, but are not limited to, methoxy, ethoxy, isopropoxy, and the like.
"(C₁-C₆)alkylene" means a linear saturated divalent hydrocarbon moiety of one to six carbon atoms or a branched saturated divalent hydrocarbon moiety of three to six carbon atoms, e.g., methylene, ethylene, 2,2-dimethylethylene, propylene, 2-methylpropylene, butylene, pentylene, and the like.
"halo-(C₁-C₈)alkyl " refers to an alkyl, as defined above, substituted with one or more halogen atoms, preferably with one to three halogen atoms. More preferred halo-(C₁-C₈)alkyl is the chloro- and fluoro-(C₁-C₈)alkyl.
"halo-(C₁-C₆)alkoxy "refers to an alkoxy, as defined above, substituted with one or more halogen atoms, preferably with one to three halogen atoms. More preferred halo-(C₁-C₆)alkoxy are the chloro- and fluoro-(C₁-C₈)alkoxy.
"(C₃-C₆)cycloalkyl" refers to a single saturated carbocyclic ring of thee to six ring carbons, such as cyclopropyl, cyclobutyl, cyclopentyl and cyclohexyl. Cycloalkyl may optionally be substituted with one or more substituents, preferably one, two or three, substituents. Preferably, cycloalkyl substituent is selected from the group consisting of (C₁-C₆)alkyl, hydroxy, (C₁-C₆)alkoxy, halo(C₁-C₆)alkyl, halo(C₁-C₆)alkoxy, halo, amino, mono- and di(C₁-C₆)alkylamino, hetero(C₁-C₆)alkyl, acyl, aryl and heteroaryl.
"Secondary amine" refers to an amine of formula HNR²R³ wherein R² and R³ may be the same or different and are independently selected from (C₁-C₆)alkyl or (C₃-C₆)cycloalkyl, or R² and R³ taken together with the nitrogen atom to which they are attached, form a (C₄-C₈) heterocycloalkane optionally containing an additional heteroatom selected from O or N. Representative examples include, but are not limited to, piperidine, 4-methyl-piperidine, piperazine, pyrrolidine, morpholine, dimethylamine, diethylamine, diisopropylamine, dicyclohexylamine, ethylmethylamine, ethylpropylamine and methylpropylamine. Preferably, the secondary amine is chosen from diethylamine, diisopropylamine, dicyclohexylamine, ethylmethylamine, ethylpropylamine, methylpropylamine and morpholine. The more preferred secondary amine is diethylamine or diisopropylamine, most preferred diethylamine.
"(C₄-C₈)heterocycloalkane" refers to a saturated non-aromatic cyclic compound of 4 to 8 ring atoms in which one or two ring atoms are heteroatoms selected from N or O, and the heterocycloalkane may be optionally substituted with one or more (C₁-C₃)alkyl, preferably one (C₁-C₃)alkyl.
"Acyl" means a group of the formula -C(O)-R^{ag}, -C(O)-OR^{ag}, -C(O)-OC(O)R^{ag} or -C(O)-NR^{ag}R^{ah} wherein R^{ag} is hydrogen, (C₁-C₆)alkyl, halo(C₁-C₆)alkyl, heteroalkyl or amino as defined herein, and R^{ah} is hydrogen or (C₁-C₆)alkyl as defined herein.
"Amino" means a group -NR^{ba}R^{bb} wherein R^{ba} and R^{bb} each independently is hydrogen or (C₁-C₆)alkyl.
"Aryl" means a monovalent monocyclic or bicyclic aromatic hydrocarbon moiety which is optionally substituted with one or more, preferably one, two or three, substituents, each of which is preferably selected from the group consisting of (C₁-C₆)alkyl, hydroxy, (C₁-C₆)alkoxy, halo(C₁-C₆)alkyl, halo(C₁-C₆)alkoxy, halo, nitro, cyano, amino, mono- and di(C₁-C₆)alkylamino, methylenedioxy, ethylenedioxy, acyl, hetero(C₁-C₆)alkyl, aryl, optionally substituted heteroaryl, optionally substituted aralkyl, and optionally substituted heteroaralkyl. A particularly preferred aryl substituent is halide. More specifically the term aryl includes, but is not limited to, phenyl, 1-naphthyl, 2-naphthyl, and the like, each of which can be substituted or unsubstituted.
"Aralkyl" refers to a moiety of the formula -R^{bc}-R^{bd} where R^{bd} is aryl and R^{bc} is (C₁-C₆)alkylene as defined herein.
"Heteroaryl" means a monovalent monocyclic or bicyclic moiety of 5 to 12 ring atoms having at least one aromatic ring containing one, two, or three ring heteroatoms selected from N, O, or S (preferably N or O), the remaining ring atoms being C, with the understanding that the attachment point of the heteroaryl moiety will be on an aromatic ring. The heteroaryl ring is optionally substituted independently with one or more substituents, preferably one, two or three substituents, each of which is independently selected from (C₁-C₆)alkyl, halo(C₁-C₆)alkyl, hydroxy, (C₁-C₆)alkoxy, halo, nitro and cyano. More specifically the term heteroaryl includes, but is not limited to, pyridyl, furanyl, thienyl, thiazolyl, isothiazolyl, triazolyl, imidazolyl, isoxazolyl, pyrrolyl, pyrazolyl, pyrimidinyl, benzofuranyl, tetrahydrobenzofuranyl, isobenzofuranyl, benzothiazolyl, benzoisothiazolyl, benzotriazolyl, indolyl, isoindolyl, benzoxazolyl, quinolyl, tetrahydroquinolinyl, isoquinolyl, benzimidazolyl, benzisoxazolyl or benzothienyl, imidazo[1,2-a]-pyridinyl, imidazo[2,1-b]thiazolyl, and the derivatives thereof.
"nitrosylating agent"comprises nitrosylsulfuric acid, sodium nitrite or a mixture thereof. Most preferably, the nirtosylating agent is nitrosylsulfuric acid.
"sulfonate ester" of R¹CH₂-OH" or (R^{ca})(R^{cb})CH-OH refers to a substituted or an unsubstituted phenyl-sulfonate, an unsubstituted naphthalene-sulfonate or a (C₁-C₆)alkylsulfonate ester derivative of R¹CH₂-OH or (R^{ca})(R^{cb})CH-OH, respectively, wherein substituted phenyl and the (C₁-C₆)alkyl chain, R¹, R^{ca}, R^{cb} are as defined herein. Representative examples include, but are not limited to, benzenesulfonic acid 2-ethyl-butyl ester, 1-naphthalenesulfonic acid 2-ethyl-butyl ester, 2-naphthalenesulfonic acid 2-ethyl-butyl ester, toluene-4-sulfonic acid 2-ethyl-butyl ester, 4-nitro-benzenesulfonic acid 2-ethyl-butyl ester, 2,4,6-trimethyl-benzenesulfonic acid 2-ethyl-butyl ester, ethanesulfonic acid 2-ethyl-butyl ester, methanesulfonic acid 2-ethyl-butyl ester and butanesulfonic acid 2-ethyl-butyl ester.
"strong acid" refers to an acid that dissociates completely in an aqueous solution with a pH ≤ 2. The strong acids include, but are not limited to: sulphuric acid (H₂SO₄), hydrohalogenic acid (i.e. HX" wherein X" is I, Br, Cl or F), nitric acid (HNO₃), phosphoric acid (H₃PO₄) and combinations thereof. Preferably, the strong acid is H₂SO₄ or hydrohalogenic acid, wherein X" is Br or Cl. Most preferably, the strong acid is H₂SO₄. Preferably the concentration of H₂SO₄ in water is in the range of 75% to 90 %, more preferably 78 to 83 %, most preferably 82.5 %.
"aqueous base" refers to a solution comprising a base and water. Numerous bases which readily dissolve in water are known in the art, such as NaOH, KOH, Ca(OH)₂, Mg(OH)₂, preferably NaOH or KOH. More preferably the aqueous base has a pH of 12 to 14.

Accordingly, in another embodiment the present invention provides a process comprising the synthetic steps represented in the following scheme 1: 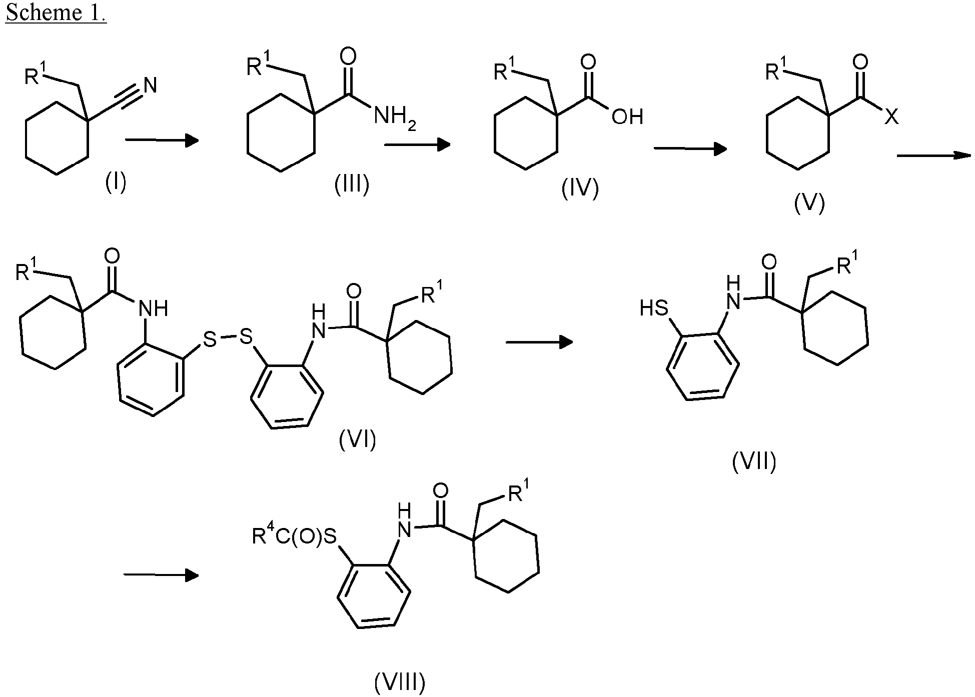 wherein X is I, Br, Cl or F, R¹ is as defined above and R⁴ is (C₁-C₈)alkyl. In particular, the process comprises hydrolysing cyclohexanecarbonitrile derivative of formula (I) to obtain a cyclohexanecarboxylic acid amide derivative of formula (III) with for example H₂O in the presence of a strong acid, or with an aqueous base. The process further comprises reacting the said cyclohexanecarboxylic acid amide derivative with a nitrosyla ting agent, to obtain the compound of formula (IV). the process comprises reacting a cyclohexanecarboxylic acid derivative of formula (IV) with a halogenating agent, such as PX₃, PX₅, SOX₂ or NCX, to obtain the acyl halide of formula (V). The halogenating step is preferably carried out in the presence of tri-(C₁-C₅)alkylamine. Furthermore, the process comprises reacting acyl halide with bis(2-aminophenyl)disulfide to acylate the amino groups of the bis(2-aminophenyl)disulfide, reducing the amino-acylated disulfide product with a reducing agent such as triphenylphosphine, zinc or sodium borohydride to yield the thiol product, and acylating the thiol group in the thiol product with R⁴C(O)X', wherein X' is I, Br, Cl or F.

The additional steps may be performed, e.g., according to the procedures described in Shinkai et al., J. Med. Chem. 43:3566-3572 (2000), WO 2007/051714, WO2009121788.

Preferably the halogenating agent is chosen from thionyl chloride, phosphorus pentachloride, oxalyl chloride, phosphorus tribromide and cyanuric fluoride, most preferably thionyl chloride. The acyl halide of formula (V) wherein X is Cl is most preferred.

In the thiol acylation step, preferably the acylating agent is R⁴C(O)X', wherein X' is Cl. Most preferably R⁴ is isopropyl.

Unless otherwise stated, organic solvent referred herein comprises ether like solvent (e.g. tetrahydrofuran, methyltetrahydrofuran, diisopropyl ether, t-butylmethyl ether or dibutyl ether, ethyl acetate, butyl acetate), alcohol solvent (e.g. methanol or ethanol), aliphatic hydrocarbon solvent (e.g. hexane, heptane or pentane), saturated alicyclic hydrocarbon solvent (e.g. cyclohexane or cyclopentane) or aromatic solvent (e.g. toluene or t-butyl-benzene)

In a further embodiment, the present invention provides processes as described above wherein nitrosylating agent is generated in situ e.g. mixing H₂SO₄ and nitrous acid (HNO₂) or H2SO₃/HNO₃ or N₂O₃/H₂SO₄ or HNO₃/SO₂ to obtain nitrosulfuric acic (NOHSO₄).

In another embodiment the invention provides a process for the preparation of the compound of a cyclohexanecarbonitrile derivative of formula (I): 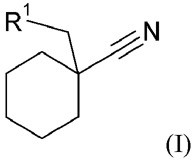 wherein R¹ is (C₁-C₈)alkyl, preferably pent-3-yl, comprising adding a Grignard reagent, such as (C₁-C₆)alkyl-magnesium-halide, phenyl-magnesium-halide, heteroaryl-magnesium-halide or (C₃-C₆)cycloakyl-magnesium-halide to a solution or mixture comprising cyclohexanecarbonitrile of formula (II), secondary amine and an alkylating agent which is 1-halo-CH₂R¹, preferably 1-halo-2-ethylbutane, or a sulfonate ester of R¹CH₂-OH, preferably of 2-ethyl-1-butanol, wherein R¹is as defined above.

Within the processes defined above, preferably the halide of Grignard reagent is chosen from chloride, bromide and iodide, more preferably chloride or bromide, most preferably chloride. The preferred alkyl of the Grignard reagent is (C₁-C₃) alkyl, more preferably methyl. The most preferred Grignard reagent is methylmagnesiumchloride.

The preferred alkylating agent is 1-halo-2-ethylbutane, most preferably 1-bromo-2-ethylbutane. The alkylation is performed in the presence of a catalytic amount of secondary amine, which is 0.01 to 0.5 equivalent of secondary amine with respect to cyclohexanecarbonitrile, most preferably 0.05 eq. The dosing time of the Grignard reagent, is preferably 0.5 to 4h, most preferably 1.5h. This addition can be carried out at temperature between 50 to 80C, in particular between 60 to 75°C. After the addition of the Grignard reagent the reaction mixture can be stirred at reflux for a time, in particular for one hour.

A nonprotic organic solvent is the preferred solvent during the alkylation, such as tetrahydrofuran, alone or in combination with another nonprotic solvent, e.g. from the group of the apolar solvents hexane, heptane, methyl tetrahydrofurane, toluene and t-butyl-benzene, more preferably hexane, heptane, toluene and t-butyl-benzene. Most preferably the nonprotic solvent is tetrahydrofuran.

Preferably the hydrolysing agent of the cyclohexanecarbonitrile derivative of formula (I) is a strong acid. The most preferred strong acid is sulphuric acid. The hydrolysis step is either carried out by dosing compound of formula (I) to sulphuric acid at a temperature of 80 °C to 120°C or both compound of formula (I) and sulphuric acid are heated as a mixture to a temperature of 80 °C to 120 °C. More preferably the temperature in both modes of addition is 95 to 110 °C, most preferably 105 to 110 °C. 1.5 to 4 equivalents of sulphuric acid with respect to compound of formula (I) is preferably used. More preferably 1.9 to 3.6 equivalents are used. Most preferably 2 equivalents are used. The hydrolysis is carried out with excess water, preferably 5 to 25 eq. of water with respect to the compound of formula (I), more preferably 10 to 20 eq. Most preferably, 14 to 16 eq. of water is used with respect to the compound of formula (I).

For the hydrolysis of the amide of formula (III), preferably 1.1 to 1.4 equivalents of nitrosylsulfuric acid is used, most preferably 1.2 to 1.4 equivalent. Either nitrosylsulfuric acid is added first and followed by water or the water is first added and followed by addition of nitrosylsulfuric acid. The second addition mode is preferred. Preferably, the dosing temperature is at 20 to 65°C, most preferably 60 to 65°C.

According to the present invention the "basic aqueous solution" for the extraction step (c) is preferably chosen from inorganic bases or organic bases, a mixture thereof, or from commonly known buffering solutions of suitable pH. The preferred inorganic base is an alkali base, such as alkali carbonate, alkali bicarbonate, alkali borate, alkali phosphate, alkali-hydroxide. A more preferred basic aqueous solution is chosen from solution of potassium bicarbonate, sodium bicarbonate, potassium carbonate, sodium carbonate, sodium borate, sodium hydroxide, or a mixture thereof. The most preferred basic aqueous solution is a solution of sodium bicarbonate, sodium hydroxide or a mixture thereof.

In a further embodiment the present invention provides a process for the preparation of [2-([[1-(2-ethylbutyl)-cyclohexyl]-carbonyl]amino)phenyl]2-methylpropanethioate comprising the formation of a compound of formula (I) obtained by any of the processes and conditions mentioned previously.

The starting materials and reagents, which do not have their synthetic route explicitly disclosed herein, are generally available from commercial sources or are readily prepared using methods well known to the person skilled in the art. For instance, compound of formula (II) is commercially available or can be prepared by procedures known to the skilled person.

The methods of the present invention may be carried out as semi-continuous or continuous processes, more preferably as continuous processes.

The following examples are provided for the purpose of further illustration and are not intended to limit the scope of the claimed invention.

The following abbreviations and definitions are used: br (broad); BuLi (butyllithium); CDCl₃ (deuterated chloroform); eq. (equivalent); g (gram); GC (gas chromatography); h (hour); HCl (hydrochloric acid); H₂O (water); HPLC (High-Performance Liquid Chromatography); ISP (Isotopic Spin Population); KOH (Potassium Hydroxide); LDA (Lithium Diisopropylamide ); M (Molar); m (multiplet); MS (Mass Spectroscopy); mL (milliliter); NaOH (Sodium hydroxide); NMR (nuclear magnetic resonance); s (singlet); sec (second); t (triplet); THF (tetrahydrofuran);

### Example 1 : 1-(2-Ethyl-butyl)-cyclohexanecarbonitrile

Under argon 50.0 g cyclohexane carbonitrile (458 mmol), 1.68g (2.39 mL) diethylamine (22.9 mmol, 0.05 eq.), 76.4 g (64.7 mL) 2-ethylbutyl bromide (463 mmol, 1.01 eq) and 101 g (114 mL) THF are added at 25°C. Then at a temperature of 70 °C using an infusion pump within 4 hours, 173 g methylmagnesiumchloride solution (3M) in THF (22.2% (m/m), 513 mmol, 1.12 eq.) are added. The reaction is stirred for 1h at reflux temperature (73°C). A conversion control sample shows <0.1 % (red. area) cyclohexanecarbonitrile. After reaction completion the temperature of the reaction mixture is reduced to 66°C. 232g (232 mL) water 24.8 g (20.6 mL) HCl 37% (251 mmol, 0.55 eq) and 62 g (91.2 mL) heptane are charged under stirring at 25°C. The above hot reaction mixture (55°C) is transferred from the reactor into the flask (25-60) within 15 minutes. The reactor is washed with 20g (23 mL) THF and the wash solvent is also transferred into the Erlenmeyer flask. The biphasic mixture is stirred for 10 minutes. The two clear phases are separated and the lower aqueous phase is removed. The upper organic phase containing product is washed with 154 g water and concentrated at 50°C / <20 mbar. The residue is degassed at 50°C/ <20mbar. Obtained are 89.4 g 1-(2-ethyl-butyl)-cyclohexanecarbonitrile crude (Assay: 93.8%, 434 mmol, yield: 94.2%) as an yellow to light brown oil. The product is transferred to a distillation flask. First the pressure in the distillation flask is reduced to 7 mbar, then 1-(2-ethyl-butyl)-cyclohexanecarbonitrile crude heated slowly to 116°C .Collected are 6.56 g 1^{st} cut (1.75g, assay: 78.8%, 2% yield) and 2^{nd} cut 4.81g, assay: 93.9%, yield 5%) as a colorless to light yellow liquid at 109-116°C) and then further cuts at 116-117°C) to give 73.6 g 1-(2-ethyl-butyl)-cyclohexanecarbonitrile distilled (assay: 98.5%, yield 82%) as a colorless liquid. Discarded are 2.0 g distillation residue as a brown liquid.

### Example 2 : 1-(2-Ethyl-butyl)-cyclohexanecarboxylic acid

Under argon 21.1 g (11.6 ml) sulfuric acid (96%) (207 mmol, 2.0 eq, contains 0.84 g water (47 mmol, 0.46 eq)) and 1.96 g water (109 mmol, 1.05 eq) is heated to 105 °C Tᵢ. 20.0 g 1-(2-ethyl-butyl)-cyclohexanecarbonitrile (103 mmol, 1.0 eq) is added within 15min at 105°C Tᵢ and the reaction mixture is stirred 2h. A conversion control sample shows 1-(2-ethyl-butyl)-cyclohexanecarbonitrile <0.1%. The reaction mixture is cooled to 50°C Tᵢ. Then 28.0 g water (1.55 mol, 15 eq) is added within 5 minutes at 51°C Tᵢ (exotherm). The reaction mixture temperature is adjusted to 61 °C Tᵢ and with vigorous stirring 36.2 g (19 mL) nitrosyl sulfuric acid (40%) in sulfuric acid (114 mmol, 1.1 eq) is added constantly within 75 minutes at 60°C Tᵢ. The reaction mixture is stirred for 45 minutes at 64 °C Tᵢ. A conversion control sample shows 0.2 norm% 1-(2-ethyl-butyl)-cyclohexanecarboxylic acid amide). To the biphasic mixture at 64 °C 20 g water is added and 13 g aq. HNOₓ is evaporated at 131-137 °C and 1000 mbar. 20 g water is added and 20 g aq. HNOₓ is evaporated at 131-137 °C and 1000 mbar. In the residue <50 ppm nitrite/nitrate are found. The reaction mixture is cooled to 20 °C 20.0 g (29.4 mL) Heptane are added and the biphasic mixture is stirred for 5 minutes. The lower aqueous phase is separated and discarded.

To the organic phase 20.0 g water is added and the biphasic mixture is stirred for 10 minutes. The lower aqueous phase is separated and discarded. 1-(2-ethyl-butyl)-cyclohexanecarboxylic acid in heptane is filtered using a paper filter and stored. The product containing organic phase is distilled in a Dean-Stark-apparatus at 112°C and 1000 mbar until no water can be removed in the water separator.

The organic phase is concentrated at 112°C and 1000 mbar to final a volume of 40 mL (27.2g) clear heptane phase. 10 g (14.7 mL) heptane are added. Obtained are 36.56 g 1-(2-ethyl-butyl)-cyclohexanecarboxylic acid in heptane (91.3 mmol, assay 53.01%, contained weight: 19.38 g 1-(2-Ethyl-butyl)-cyclohexanecarboxylic acid, yield 88.2%) as light yellow to orange solution.

### Example 3 : 1-(2-Ethyl-butyl)-cyclohexanecarbonitrile

To a 1-litre jacketed flask fitted with a stirrer, thermometer, condenser and pressure-equalised dropping funnel and purged with nitrogen were added cyclohexanecarbonitrile (21.8 g, 200 mmol), diethylamine (1.46 g, 20 mmol), 2-ethylbutybromide (33.3 g, 202 mmol) and tetrahydrofuran (44.0 g). The resulting clear solution was heated to 45 °C and stirred under a continuous stream of nitrogen. Methylmagnesium chloride in tetrahydrofuran (83 g of a 22% solution, 0.246 mmol) was added over one hour while maintaining the temperature of the reaction mixture between 45.3 and 61.4 °C. The mixture was then refluxed between 67.4 and 70.2 °C for 75 minutes. Analysis of the reaction mixture by GLC showed 98.1% 1-(2-ethyl-butyl)-cyclohexanecarbonitrile, 0.9% ethylbutylbromide, 0.0% cyclohexanecarbonitrile and 0.2% acetylcyclohexane. The mixture was cooled to 48.7 °C then transferred over 25 minutes into a stirred mixture of deionised water (101 g), hydrochloric acid (37%, 10.8 g) and n-heptane (27 g) which had been precooled to 15 °C. The temperature was kept between 15 and 60 °C during the addition. The reaction flask was rinsed with tetrahydrofuran (8.9 g) into the quenched mixture which was then cooled and agitated at between 15 and 30 °C for 20 minutes. After settling for 10 minutes the lower aqueous layer was split off. The remaining organic layer was washed with deionised water (68 g) before being concentrated under reduced pressure on the rotary evaporator at up to 60 °C until no further solvent distilled over. The product was further degassed under high vacuum at 80 °C to leave 38.3 g of pale yellow oil. The w/w assay of the product as determined by internal standard GLC was 95.8%, giving a contained yield of 1-(2-ethyl-butyl)-cyclohexanecarbonitrile of 36.7 g or 95.0% of theory. Area normalised assay by GLC showed 1-(2-ethyl-butyl)-cyclohexanecarbonitrile 99.1%, ethylbutyl bromide 0.2%, acetylcyclohexane 0.2% and others 0.5%.

### Example 4 : 1-(2-Ethyl-butyl)-cyclohexanecarbonitrile

To a 1-litre jacketed flask fitted with a stirrer, thermometer, condenser and pressure-equalised dropping funnel and purged with nitrogen were added cyclohexanecarbonitrile (21.8 g, 200 mmol), diethylamine (0.37 g, 20 mmol), 2-ethylbutyl bromide (33.3 g, 202 mmol) and tetrahydrofuran (44.0 g). The resulting clear solution was heated to 45 to 50 °C and stirred under a continuous stream of nitrogen. Methylmagnesium chloride in tetrahydrofuran (83 g of a 22% solution, 0.246 mmol) was added over 65 minutes while maintaining the temperature of the reaction mixture between 46.0 and 55.2 °C. The mixture was then refluxed between 67.5 and 70.2 °C for 100 minutes. Analysis of the reaction mixture by gas liquid chromatography (GLC) showed 96.6%1-(2-ethyl-butyl)-cyclohexanecarbonitrile, 2.0% ethylbutylbromide, 0.0% cyclohexanecarbonitrile and 0.9% acetylcyclohexane. The mixture was cooled to around 50 °C then transferred over 15 minutes into a stirred mixture of deionised water (101 g), hydrochloric acid (37%, 10.8 g) and n-heptane (27 g) which had been precooled to 15 °C. The temperature was kept between 15 and 60 °C during the addition. The reaction flask was rinsed with tetrahydrofuran (8.9 g) into the biphasic mixture which was then cooled and agitated at between 15 and 30 °C for 20 minutes. After settling for 10 minutes the lower aqueous layer was split off. The remaining organic layer was washed with deionised water (68 g) before being concentrated under reduced pressure on the rotary evaporator at up to 50 °C until no further solvent distilled over. The product was further degassed under high vacuum at 80 °C to leave 37.7 g of pale yellow oil. The w/w assay of the product as determined by internal standard gas liquid chromatography (GLC) was 96.9%, giving a contained yield of 1-(2-ethyl-butyl)-cyclohexanecarbonitrile of 36.5 g or 94.6% of theory. Area normalised assay by GLC showed 1-(2-ethyl-butyl)-cyclohexanecarbonitrile 97.9%, ethylbutyl bromide 0.8%, acetylcyclohexane 1.1% and others 0.2%.

## Claims

1. A process for the preparation of the compound of a cyclohexanecarbonitrile derivative of formula (I): 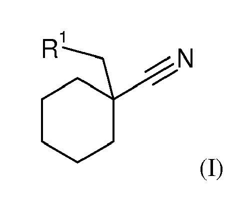 wherein R¹ is (C₁-C₈)alkyl, comprising adding a Grignard reagent to cyclohexanecarbonitrile of formula (II) 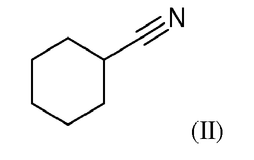 in the presence of an alkylating agent, wherein the alkylating agent is 1-halo-CH₂R¹ or a sulfonate ester of R¹CH₂-OH,
wherein the coupling reaction is carried out in the presence of a secondary amine
wherein 0.01 to 0.5 equivalents of the secondary amine with respect to cyclohexanecarbonitrile is used.

2. A process according to claim 1, for the preparation of the compound of a cyclohexanecarbonitrile derivative of formula (I): 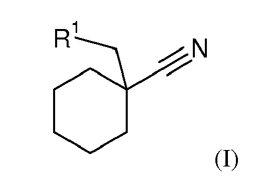 wherein R¹ is (C₁-C₈)alkyl,
comprising adding a Grignard reagent to a solution or mixture comprising cyclohexanecarbonitrile of formula (II) according to claim 1, secondary amine and an alkylating agent.

3. A process according to anyone of claims 1 to 2 further comprising the preparation of a cyclohexanecarboxylic acid derivative of formula (IV): 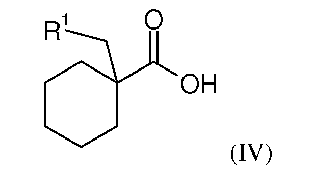 wherein R¹ is as defined in claim 1, comprising:
a) hydrolysing a cyclohexanecarbonitrile derivative of formula (I): 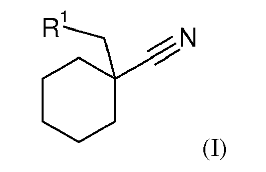 to obtain a cyclohexanecarboxylic acid amide derivative of formula (III); 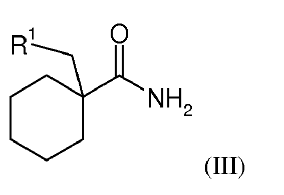
b) further hydrolysing compound of formula (III) to obtain the compound of formula (IV).

4. A process according to claim 3 additionally comprising the step of reacting a halogenating agent in the presence of a tri-(C₁-C₅)alkylamine with compound of formula (IV) as defined in claim 3, to obtain compound of formula (V), wherein X is I, Br, Cl or F:

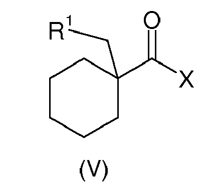

5. The process according to claim 4, further comprising the step of acylating a compound of the formula VI' 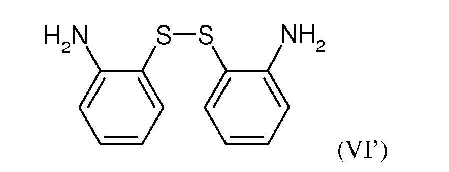 with a compound of formula (V) to obtain a compound of formula VI: 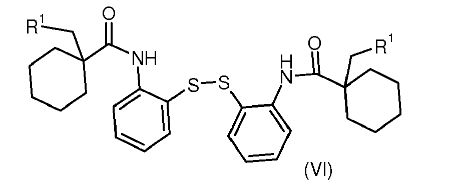 wherein R¹ is as defined in claim 1.

6. The process according to claim 5 further comprising the step of reducing the compound of formula VI with a reducing agent to obtain a compound of formula VII :

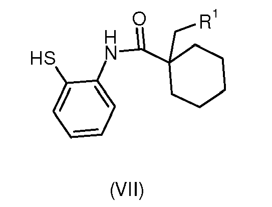

7. The process according to claim 6 further comprising the step of acylating the compound of formula VII with R⁴C(O)X', wherein X' is I, Br, Cl or F, to obtain a compound of formula VIII: 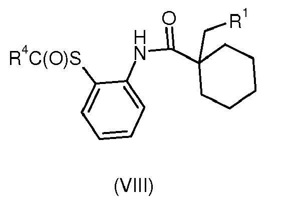 wherein R⁴ is (C₁-C₈)alkyl and R¹ is as defined in claim 1.

8. A process according to any one of claims 1 or 2, wherein the coupling reaction is followed by a mineral acid quenching, such as hydrofluoric acid, hydrochloric acid, boric acid, acetic acid, formic acid, nitric acid, phosphoric acid or sulfuric acid.

9. A process according to any one of claims 1, 2 or8, wherein the coupling reaction is followed by a hydrochloric acid quenching.

10. A process according to any one of claims 1 to 9, wherein the alkylating agent is 1-bromo-2-ethylbutane.

11. A process according to any one of claims 1 to 10, wherein the Grignard reagent is methylmagnesiumchloride.

12. A process according to any one of claims 1 to 11, wherein the secondary amine is diethylamine.

13. A process according to claim 1, wherein R¹ is pent-3-yl.

## Patentansprüche

1. Ein Verfahren zur Herstellung der Verbindung von einem Cyclohexancarbonitrilderivat der Formel (I): 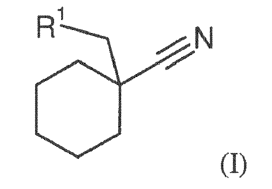 wobei R¹ gleich (C₁-C₈)Alkyl ist, umfassend die Zugabe eines Grignard-Reagens zu Cyclohexancarbonitril der Formel (II): 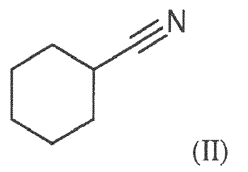 in Gegenwart eines Alkylierungsmittels, wobei das Alkylierungsmittel 1-Halo-CH₂R¹ oder ein Sulfonatester von R¹CH₂-OH ist,
wobei die Kupplungsreaktion in Gegenwart eines sekundären Amins durchgeführt wird, wobei 0,01 bis 0,5 Äquivalente von dem sekundären Amin in Bezug auf das Cyclohexancarbonitril verwendet werden.

2. Ein Verfahren gemäß Anspruch 1 zur Herstellung der Verbindung von einem Cyclohexancarbonitrilderivat der Formel (I): 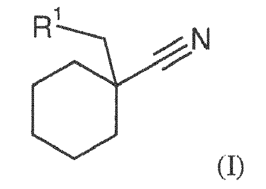 wobei R¹ gleich (C₁-C₈)Alkyl ist,
umfassend die Zugabe eines Grignard-Reagens zu einer Lösung oder einem Gemisch umfassend Cyclohexancarbonitril der Formel (II) gemäß Anspruch 1, sekundäres Amin und ein Alkylierungsmittel.

3. Ein Verfahren gemäß einem der Ansprüche 1 bis 2 weiter umfassend die Herstellung eines Cyclohexancarbonsäurederivats der Formel (IV): 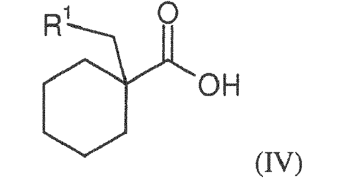 wobei R¹ wie in Anspruch 1 definiert ist, umfassend:
a) Hydrolisieren eines Cyclohexancarbonitrilderivats der Formel (I): 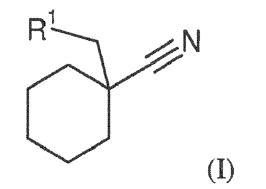 um ein Cyclohexancarbonsäureamidderivat der Formel (III) zu erhalten; 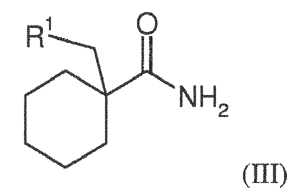
b) weiter Hydrolisieren der Verbindung der Formel (III), um die Verbindung der Formel (IV) zu erhalten.

4. Ein Verfahren gemäß Anspruch 3, zusätzlich umfassend den Schritt der Umsetzung eines Halogenierungsmittels in Gegenwart eines Tri-(C₁-C₅)alkylamins mit Verbindung der Formel (IV) wie in Anspruch 3 definiert, um Verbindung der Formel (V) zu erhalten, wobei X gleich I, Br, Cl oder F ist:

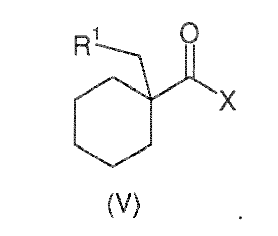

5. Das Verfahren gemäß Anspruch 4, weiter umfassend den Schritt der Acylierung einer Verbindung der Formel VI': 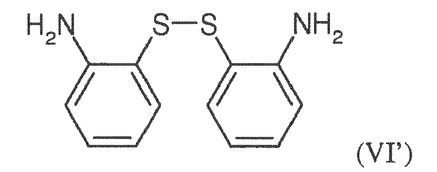 mit einer Verbindung der Formel (V), um eine Verbindung der Formel VI zu erhalten: 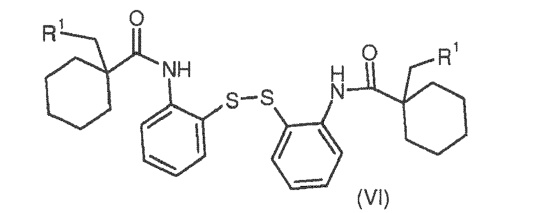 wobei R¹ wie in Anspruch 1 definiert ist.

6. Das Verfahren gemäß Anspruch 5, weiter umfassend den Schritt der Reduktion der Verbindung der Formel VI mit einem Reduktionsmittel, um eine Verbindung der Formel VII zu erhalten:

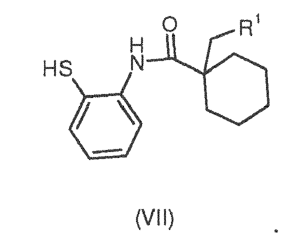

7. Das Verfahren gemäß Anspruch 6, weiter umfassend den Schritt der Acylierung der Verbindung der Formel VII mit R⁴C(O)X', wobei X' gleich I, Br, Cl oder F ist, um eine Verbindung der Formel VIII zu erhalten: 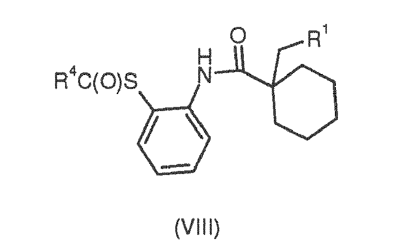 wobei R⁴ gleich (C₁-C₈)Alkyl ist und R¹ wie in Anspruch 1 definiert ist.

8. Ein Verfahren gemäß einem der Ansprüche 1 oder 2, wobei der Kupplungsreaktion ein Mineralsäurequenchen, wie Fluorwasserstoffsäure, Salzsäure, Borsäure, Essigsäure, Ameisensäure, Salpetersäure, Phosphorsäure oder Schwefelsäure, folgt.

9. Ein Verfahren gemäß einem der Ansprüche 1, 2 oder 8, wobei der Kupplungsreaktion ein Salzsäurequenchen folgt.

10. Ein Verfahren gemäß einem der Ansprüche 1 bis 9, wobei das Alkylierungsmittel 1-Brom-2-ethylbutan ist.

11. Ein Verfahren gemäß einem der Ansprüche 1 bis 10, wobei das Grignard-Reagens Methylmagnesiumchlorid ist.

12. Ein Verfahren gemäß einem der Ansprüche 1 bis 11, wobei das sekundäre Amin Diethylamin ist.

13. Ein Verfahren gemäß Anspruch 1, wobei R¹ gleich Pent-3-yl ist.

## Revendications

1. Procédé pour la préparation du composé d'un dérivé de cyclohexanecarbonitrile de formule (I) : 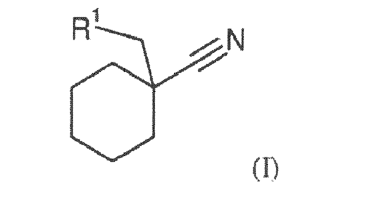 dans laquelle R¹ représente un groupe alkyle en C₁ à C₈, comprenant l'addition d'un réactif de Grignard à du cyclohexanecarbonitrile de formule II 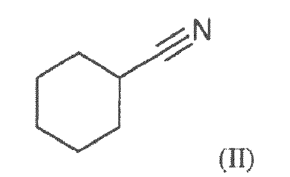 en présence d'un agent alkylant, ledit agent alkylant étant un agent de formule 1-halogéno-CH₂R¹ ou un ester sulfonate de formule R¹CH₂-OH,
dans lequel la réaction de couplage est effectuée en présence d'une amine secondaire,
une quantité de 0,01 à 0,5 équivalent de l'amine secondaire par rapport au cyclohexanecarbonitrile étant utilisée.

2. Procédé suivant la revendication 1, pour la préparation du composé d'un dérivé de cyclohexanecarbonitrile de formule (I) : 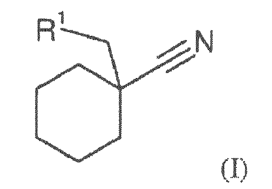 dans laquelle R¹ représente un groupe alkyle en C₁ à C₈, comprenant l'addition d'un réactif de Grignard à une solution ou un mélange comprenant le cyclohexanecarbonitrile de formule (II) suivant la revendication 1, une amine secondaire et un agent alkylant.

3. Procédé suivant l'une quelconque des revendications 1 et 2, comprenant en outre la préparation d'un dérivé d'acide cyclohexanecarboxylique de formule (IV) : 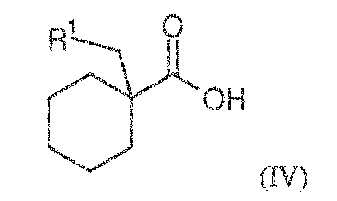 dans laquelle R¹ est tel que défini dans la revendication 1, comprenant :
a) l'hydrolyse d'un dérivé de cyclohexanecarbonitrile de formule (I) : 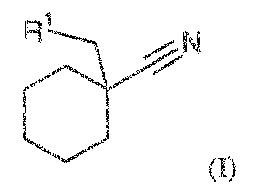 pour obtenir un dérivé d'amide d'acide cyclohexanecarboxylique de formule (III) : 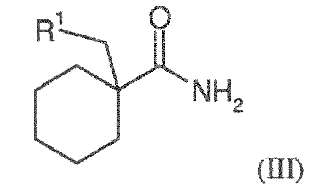
b) en outre l'hydrolyse du composé de formule (III) pour obtenir le composé de formule (IV).

4. Procédé suivant la revendication 3, comprenant en outre l'étape de réaction d'un agent d'halogénation en présence d'une tri-(alkyle en C₁ à C₅)-amine avec un composé de formule (IV) tel que défini dans la revendication 3, pour obtenir un composé de formule (V), dans lequel X représente un groupe I, Br, Cl ou F :

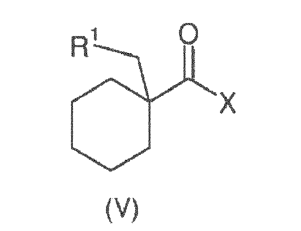

5. Procédé suivant la revendication 4, comprenant en outre l'étape d'acylation d'un composé de formule VI' 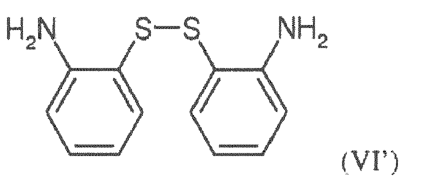 avec un composé de formule (V) pour obtenir un composé de formule VI : 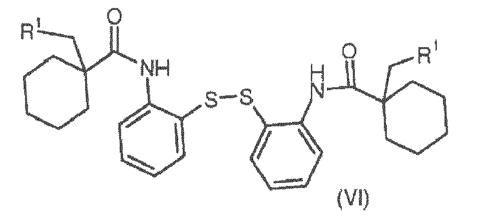 dans laquelle R¹ est tel que défini dans la revendication 1.

6. Procédé suivant la revendication 5, comprenant en outre l'étape de réduction du composé de formule VI avec un agent réducteur pour obtenir un composé de formule VII :

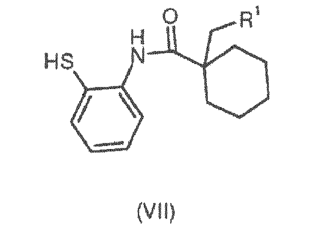

7. Procédé suivant la revendication 6, comprenant en outre l'étape d'acylation du composé de formule VII avec un composé de formule R⁴C(O)X', dans laquelle X' représente un groupe I, Br, Cl ou F, pour obtenir un composé de formule VIII : 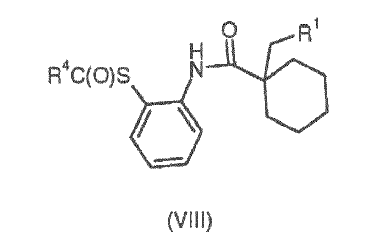 dans laquelle R⁴ représente un groupe alkyle en C₁ à C₈ et R¹ est tel que défini dans la revendication 1.

8. Procédé suivant l'une quelconque des revendications 1 et 2, dans lequel la réaction de couplage est suivie par une désactivation avec un acide minéral tel que l'acide fluorhydrique, l'acide chlorhydrique, l'acide borique, l'acide acétique, l'acide formique, l'acide nitrique, l'acide phosphorique ou l'acide sulfurique.

9. Procédé suivant l'une quelconque des revendications 1, 2 et 8, dans lequel la réaction de couplage est suivie par une désactivation avec de l'acide chlorhydrique.

10. Procédé suivant l'une quelconque des revendications 1 à 9, dans lequel l'agent alkylant est le 1-bromo-2-éthylbutane.

11. Procédé suivant l'une quelconque des revendications 1 à 10, dans lequel le réactif de Grignard est le chlorure de méthylmagnésium.

12. Procédé suivant l'une quelconque des revendications 1 à 11, dans lequel l'amine secondaire est la diéthylamine.

13. Procédé suivant la revendication 1, dans lequel R¹ représente un groupe pent-3-yle.
